# EUROPEAN PATENT SPECIFICATION

(11) **EP 0 876 204 B1**
(45) Date of publication and mention of the grant of the patent: **22.09.2004**
(21) Application number: 95944490.2
(22) Date of filing: 06.12.1995
(51) Int. Cl.: A61K 7/15, B01J 13/00, A61L 9/04, A61L 9/00

(54) **POST-FOAMABLE FOAM COMPOSITION**
VERZÖGERT SCHÄUMENDE SCHAUMZUSAMMENSETZUNG
COMPOSITION MOUSSANTE A MOUSSAGE DIFFERE

(43) Date of publication of application: 11.11.1998
(73) Proprietor: S.C. JOHNSON & SON, INC., Racine, Wisconsin 53403 (US)
(72) Inventor: Monson, James A, Dousman, WI 53118 (US)
(74) Representative: Carpmaels & Ransford
(86) International application number: PCT/US1995/016475
(87) International publication number: WO 1997/020626

(56) References cited:
- WO-A-94/02109
- GB-A- 1 444 334
- US-A- 2 665 480
- US-A- 3 072 487
- US-A- 3 541 581
- US-A- 3 970 584
- US-A- 4 122 159
- US-A- 4 652 389

## Description

### Field of the Invention

This invention is related generally to foamable compositions and, more particularly, to compositions which dispense as foams and post-foam, thereby exhibiting multiple levels of foam.

### Background of the Invention

Conventional personal care and household and industrial cleaning products are available in solid, powder, liquid gel and cream form. The most common and familiar personal care and cleaning product on the market today is a bar soap, which produces a lather or foam by agitation with the hands and body. Bar soaps come in a variety of types and are relatively inexpensive. However, problems associated with bar soaps are numerous. One of the most common problems is the difficulty in "working up" a lather from the bar for spreading. Considerable time and dexterity are usually required. Another problem associated with bar soaps is the inability to maintain sanitary conditions while exposing the bar to multiple uses and multiple users. A bar soap is often viewed as an object used by an entire household, office or other users of a common bathroom or sink. While personal hygiene is just that, personal, soap is considered a "community object". An additional problem associated with bar soaps is maintaining them in a fresh, appealing condition. Soap bars sit in soap dishes and are exposed to moisture where they tend to break down into an unsightly gelatinous state. Additionally, bar soaps tend to cause unsightly residue or "scum" on sink, bath tub and shower surfaces if exposed to water.

In an effort to overcome some of these problems, the industry developed liquid cleaning and personal care products. These products are generally available as thick liquids, such as shampoos, conditioners, shower gels, liquid soaps and cleaners. These products are relatively slow-foaming and produce very little, relatively weak foam which quickly flattens. Additionally, to maintain acceptable levels of foam, repeated use of the products in one grooming or cleaning session is often required. Further, many of these liquid or gel products are subject to the drip or drool factor, whereby, after dispensing, a portion of the substance remains in the valve of the container and tends to drool after time leaving an unsightly mess on dispensers and sink, counter and shower surfaces.

Post-foaming gel compositions and foaming compositions were developed in an effort to overcome some of the shortcomings of bar and liquid products. These compositions are typically dispensed from aerosol or barrier packages/containers. In the case of a post-foaming gel, when the gel is spread over the skin, hair or other surface and rubbed, the gel post-foams into a lathered product. One disadvantage associated with these products is that when the compositions are dispensed in the form of a stable gel, the gel is not easily spread over the surface to be treated or cleaned in an even and fluid manner. Rather, these post-foaming gel compositions tend to clump and fall off the surface to be cleaned, particularly surfaces that are not horizontal.

US 4,652,389 describes a carpet cleaning composition comprising a foam-forming surfactant, a volatile organic solvent, a propellant and water.

US 3,970,584 describes an aerosol package, suitable for dispensing shaving cream, containing a foam-forming oil-in-water emulsion and a propellant system consisting essentially of nitrous oxide and isobutane.

US 4,122,159 describes a aerosol foam composition containing a liquified gas and nitrous oxide propulsive agent, a tensio-active amphoteric foaming agent and a tertiary amino acid.

US 3,541,581 describes a cleansing or cosmetic composition consisting essentially of water, water-soluble soap, a volatile liquid post-foaming agent and a water-soluble gelling agent.

GB 1,444,334 describes a stable post-foaming gel comprising 75-90% by weight water, 5-15% by weight water soluble soap, 0.5-5% by weight volatile post-foaming agent selected from saturated aliphatic hydrocarbons, halogenated hydrocarbons, and mixtures thereof, and 0.5-8% by weight of at least one water-soluble hydroxyalkyl cellulose or polyoxyalkylene gelling aid. The gel foams spontaneously to a rich, creamy lather when it is rubbed over the user's skin.

WO 94/02109 describes self-foaming liquid cleansing compositions which are dispensed as a liquid and foams spontaneously on spreading.

Both the post-foaming gel and foam products are generally packaged in rigid pressurized aerosol containers or barrier packages/containers with hydrocarbon propellant gases contained therein. Such aerosol containers are expensive to manufacture and ship. Further, the propellants used often do not form an integral part of the composition and are typically compartmentalized from the product to provide the positive pressure needed to aid in the dispensing of the product. Such containers are known in the industry as barrier packages because they provide a barrier between the extraneous propellants and the composition to be dispensed. Further, the propellent gases released to the atmosphere by use of these containers have increasingly come under attack because they are environmentally unacceptable. Also, the containers are not readily reusable or recyclable.

Each of the prior art products suffer from various deficiencies. However, one of the greatest deficiencies is lack of foam or difficulty in obtaining and maintaining adequate foam levels during use. In personal care and household and industrial cleaning, foam and foam stability are associated with cleaning ability. The consumer equates a greater foam level with better cleaning ability. Additionally, in personal care, foams provide a particularly pleasing effect or feel if a rich, slightly wet, creamy foam can be achieved. These foam properties generally require a foam with a fine bubble texture. In the past, it has been found that the foam obtained upon being dispensed from an aerosol package is either too wet and runny with limited foam stability, or too stiff and dry, neither of which provide the essential pleasing feel or texture. Therefore, it would be an improvement in the art to provide a composition that readily produces an initial rich foam or lather and maintains suitable foam levels during the cleansing process.

In summary, a considerable number of deficiencies exist in the art relating to utilitarian compositions and in particular personal care and cleaning compositions. While the bar soaps of the prior art are inexpensive and convenient they are lacking in areas of hygiene and sanitation as well as foam production and maintenance and require dexterity of the user. Although the liquid, gel and foam products of the prior art provide for more convenience and sanitation, they also lack the optimum foam producing and foam maintaining capabilities and spreading ability desired in such personal care and cleaning compositions. Additionally, these prior art liquid, gel and foam compositions prove difficult and expensive to package and dispense. Further, these packages or containers raise concerns from an environmental standpoint because of various propellants used to dispense the composition and disposal of the containers. All of these factors further increase overall costs. Thus, there is an ongoing search for compositions which can be easily spread, provide the desired foam characteristics, while maintaining sufficient foam levels for use, and are packaged and used in a more environmentally acceptable manner. Clearly, there is a need for improved and novel personal care and cleaning compositions that provide a desired creamy, rich foam, maintain foam levels during cleaning, are economical and easily dispensed and can withstand multiple uses and multiple users while maintaining sanitary conditions and a clean, pleasing appearance.

### Objects of the Invention

It is an object of this invention to provide personal care and cleaning compositions which overcome some of the problems and shortcomings of the prior art.

Another object of this invention is to provide personal care and cleaning compositions which produce adequate initial foam and maintain foam levels during cleaning.

A further object of the invention is to provide a composition that can be used multiple times by multiple users while maintaining sanitary conditions.

Still another object of the invention is to provide a composition that is easily dispensed in a manner not harmful to the environment.

These and other important objects will be apparent from the following description and from the drawings.

### Summary of the Invention

This invention includes novel foamable compositions. It overcomes certain well-known problems and deficiencies of the prior art, including those outlined above. An important aspect of this invention is the ability to provide a composition which is dispensed as a foam and post-foams.

The inventive composition according to claim 1 exhibits multiple levels or stages of foam and provides for extended foam character, throughout the cleaning process.

The foamable utilitarian constituent includes a surface active agent selected from the group consisting of nonionic, anionic, amphoteric and cationic surfactants. The specific surface active agents used are dependent on the purpose for which the composition is to be utilized. The compositions include about 0.1 - 60.0% of the utilitarian constituent. Further, the foamable utilitarian constituent can include more than one surface active agent to impart various characteristics to the composition.

The post-foaming agent is responsible for further foaming the foam after the composition is dispensed. The post-foaming agent is about .5 - 24.0% of the novel post-foamable foam composition.

The compressed gas of the composition provides for both dispensing of the composition and initial foaming of the composition when dispensed. The compressed gas is one of the group including (a) nitrogen, (b) argon, (c) neon, (d) krypton, (e) xenon, (f) helium, (g) carbon dioxide, (h) nitrous oxide, and (i) mixtures thereof. Carbon dioxide and nitrous oxide and mixtures are preferable because they are inexpensive and easily accessible. The compressed gas is present in amounts sufficient to provide the composition, when contained in a dispenser, with dispensing pressure. The compressed gas is also present in amounts sufficient to immediately foam the composition when it is dispensed. To provide both of these functions, the compressed gas is present in amounts sufficient to provide about 169 to 514 kPa (10 - 60 pounds per square inch gauge (psig)) on the composition when it is contained.

The composition further includes a diluent. The diluent is water. The diluent assists in the foaming and lathering and is used in quantities sufficient to obtain the desired viscosity and concentration for the composition. The composition includes about 16.0 - 99.4% diluent. In preferred embodiments about 55.0 - 98.5% of the composition is diluent. Further, additives such as fragrances, emollients, thickeners, preservatives etc. can also be included in the inventive composition.

The composition is dispensed by the compressed gas and exhibits multiple sequential stages of foam. These stages of foam include an initial stage of foam where the utilitarian constituent is foamed by the compressed gas on dispensing. This initial stage of foam has a density of about 0.15 - 0.90 g/cc. The second stage of foam results from the vaporizing post-foaming agent. The second stage of foam has a density of about 0.04 - 0.30 g/cc. Additional stages of foam are possible including a stage of foam resulting from agitation of the composition on a surface to be cleaned, thereby entraining atmospheric air in the utilitarian constituent. These multiple stages or levels of foam provide for extended foam character.

### Brief Description of the Drawings

FIGURE 1 is a perspective view of an exemplary dispensing container having the inventive post-foaming composition used for bathing or the like therein.
FIGURE 2 is an elevation view of the dispensing container of FIGURE 1 shown in conjunction with a concentrate container having the concentrate charged into the dispensing container of FIGURE 1.
FIGURE 3 is a cross-sectional elevation view showing mating female and male valve stems used respectively with the upright and inverted containers shown in FIGURE 2.
FIGURE 4 is a cross-sectional elevation view of a dispensing container like that of FIGURE 1 which is free of a removable cap and has a dispensing valve made integrally therewith. Parts are broken away.
FIGURE 5 is a cross-sectional elevation view of another dispensing container like that of FIGURE 1 which includes a removable cap having a dispensing valve therewith.
FIGURE 6 is a side elevation view of an exemplary dispensing container like that of FIGURE 1 shown in conjunction with a container mounting rack as for a shower or bath, for example.
FIGURE 7 is a cross-sectional side elevation view of an exemplary dispensing container like that of FIGURE 1 shown in conjunction with an enclosed container holder for a shower or bath and having a removable top.

### Detailed Descriptions of the Preferred Embodiments

The present invention is directed to novel post-foamable foam compositions according to claim 1.

The foamable utilitarian constituent is the main ingredient of the composition which provides the function or purpose of the composition. It can be food based as in the area of dairy products, such as cream, or can be a cheese product such as cheese spread. The utilitarian constituent includes at least one surface active agent, as in the case of personal care products, such as shampoos, cleansers, hand and body cleaners and conditioners, or household and industrial cleaners, such as hard surface cleaners, multi-purpose cleaners, bathroom cleaners and glass cleaners. These surface active agents include nonionic, anionic, amphoteric and cationic surfactants. When a surface active agent is used alone, nonionic, anionic, or amphoteric surfactants are preferable. When the foamable utilitarian constituent includes a mixture of more than one surface active agent, anionic, nonionic, cationic and amphoteric surfactants can be used. Numerous surface active agents or surfactants are known and suitable for use in the composition of the present invention, depending on the function of the composition. A variety of these surface active agents can be found in McCutcheon's Emulsifiers and Detergents, 1994. An example of just a few of the numerous suitable anionic surface active agents include: alkyl benzene sulfonates in which the alkyl group contains from 9 to 15 carbon atoms, preferably 11 to 14 carbon atoms in straight chain or branches chain configuration; alkyl sulfates obtained by sulfating an alcohol having 8 to 22 carbon atoms, preferably 12 to 16 carbon atoms (the alkyl sulfates have the formula (ROSO₃)₂M where R is the C₈₋₂₂ alkyl group and M is the alkaline earth metal); paraffin sulfonates having 8 to 22 carbon atoms, preferably 12 to 16 carbon atoms in the alkyl moiety; olefin sulfonates having 8 to 22 carbon atoms, preferably 12 to 16 carbon atoms; alkyl ether sulfates derived from ethoxylating an alcohol having 8 to 22 carbon atoms, preferably 12 to 16 carbon atoms with 1 to 30, preferably 1 to 12 moles of ethylene oxide and then sulfating; and alkyl glyceryl ether sulfonates having 8 to 22 carbon atoms, preferably 12 to 16 carbon atoms in the alkyl moiety. Examples of some preferred surface active agents suitable for use in body and hand cleaners include: ammonium lauryl sulfate, decyl polyglucose, sodium laureth sulfate, decyl polyglucose, ammonium cocoyl isethioniate, and soyamidopropyl betaine. The inventive composition preferably includes about 0.10 - 60.0% foamable utilitarian constituent.

The post-foaming agent included in the composition acts as a foaming aid for the composition after the composition is dispensed as a foam. This provides for lasting foam character of the composition and multiple levels or stages of foam, thereby reducing or eliminating the need for multiple applications of the composition during cleaning to maintain foam. The post-foaming agent in the inventive compositions is a mixture of isobutane and isopentane. This mixture is useful for providing the particular vapor pressure desired. An advantage of using mixtures of two or more post-foaming agents is that although the individual agents may have vapor pressures outside the desired range, when combined in the composition, the resulting composition has a vapor pressure within the range of from 41,4 to 96,5 kPa (6 to 14 psig) at a temperature from 32-38°C (90 to 100°F). This temperature range is the most suitable for personal care products which are used on humans having a body temperature of about 37°C (98.6°F). The mixture can include 10.0 - 90.0% of iso-butane and 90.0 - 10.0% of iso-pentane. The preferred ratio of the mixture is about 3:1 or 4:1. The post-foaming agent comprises about .50 - 24.0% of the total post-foaming foam composition.

The compressed gas of the inventive composition provides two functions. Initially, the compressed gas provides the necessary dispensing pressure required to dispense the composition when contained. The second function of the compressed gas in the composition is to provide the initial level or stage of foam to the composition immediately on dispensing. To meet the above requirements, the compressed gas should be present in amounts sufficient to provide about 169-514 kPa (10 - 60 psig) on the composition when it is contained. In a preferred embodiment, the compressed gas exerts about 341 kPa (35 psig) on the composition when it is contained.

The compressed gas is one of the group including: (a) nitrogen, (b) argon, (c) neon, (d) krypton, (e) xenon, (f) helium, (g) carbon dioxide, (h) nitrous oxide, and (i) mixtures thereof.

The inventive composition also includes a diluent. The diluent is included to assist in foaming and lathering. Additionally, the diluent is used in quantities sufficient to obtain the desired viscosity and concentration for the composition. The diluent is water. The inventive composition includes about 16.0 - 99.4% diluent. In preferred embodiments about 55.0 - 98.5% of the composition is diluent.

The composition of the present invention may also contain minor amounts of conventional additional ingredients to impart various desired characteristics to the composition. Suitable additives include, for example, thickening agents, coloring agents, perfumes, preservatives, antiseptic agents, antibacterial agents, disinfectants, emollients and humectant. The composition may also optionally contain suspending agents or thickening agents for imparting desired viscosity to the composition. Suitable thickening agents include, for example, carboxy vinyl polymers available from B.F. Goodrich Company under the trademark CARBOPOL, carbomers, sodium polacrylate, hydroxyethyl cellulose, guar gum and xanthum gum. Further, the inventive compositions can include conditioning agents such as glycerine, guar hydroxypropyl trimonium chloride, fatty acid esters, and highly branched hydrocarbons such as those sold by The Permethyl Corporation under the trademark PERMETHYL 104A.

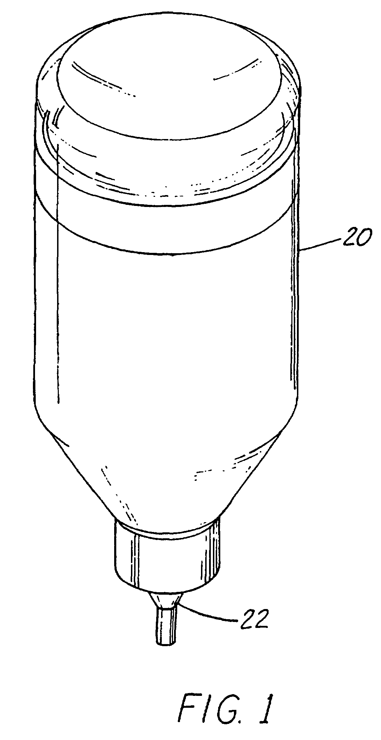

The compositions of the present invention are typically dispensed from a container having a valve arrangement. One embodiment of such a container is shown in FIGURE 1. This container 20 should be of barrier material to prevent loss of pressure. The barrier material may be a barrier plastic material, metal, glass, or a metal-coated plastic material, or laminated material. In preferred embodiments, the container 20 is translucent or transparent for reasons which will become apparent hereinafter. A preferred material for the container 20 is polyethyleneterepthalate (PET).

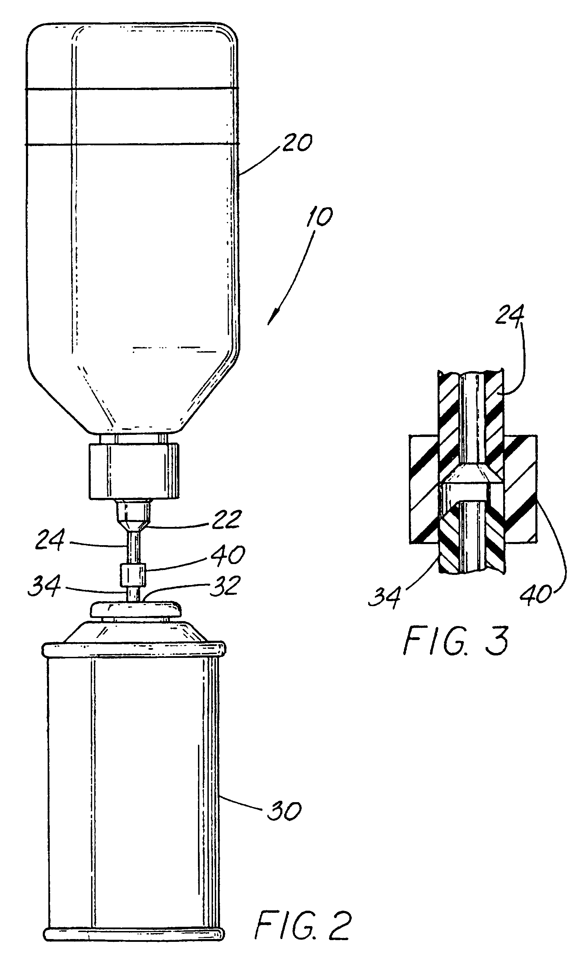

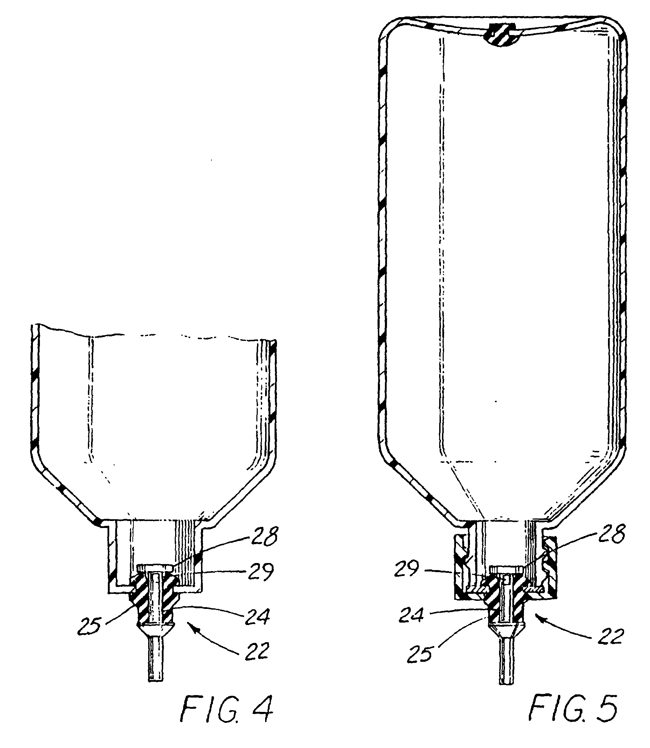

The compositions of the present invention can be prepared using various methods. In one embodiment, shown in FIGURE 2, the composition is prepared using a novel dispenser system to prepare and dispense the composition. In this embodiment, a first or dispensing container 20 having a valve 22 is provided. As shown in FIGURES 4 and 5, the valve 22 includes a hollow stem 24, a resilient stem retainer 25, a valving member 28 and inlet passages 29 in communication with the hollow passage through the stem. A diluent such as water is in the first container.

A second or concentrate container 30 having a valve 32 is also provided. The valve 32 is similar in arrangement to the valve 22 and also includes a stem 34. This second container 30 includes a concentrate. The concentrate typically includes about 2.0 - 60.0% foamable utilitarian constituent, 6.0 - 24.0% post-foaming agent and about 16.0 - 72.0% diluent. The second container 30 can be any known container including barrier packages known to one of ordinary skill in the art. The second container should include a propellant capable of dispensing the concentrate from the second container 30. The propellant should be present in such amounts to provide about 50 - 80 psig on the concentrate when contained. Any of a variety of propellants can be used so long as the terminal pressure (pressure exerted on the remaining concentrate when the second container is almost empty) is at least about 238 kPa (20 psig). Suitable propellants include the condensable gases including hydrocarbons such as propane, butane, isobutane, and isopentane. Environmentally hazardous halogenated hydrocarbons represented by the structural formula CₙHₓCl_{y}F_{z}, wherein n is a whole number from 1 to 2 and x,y and z is equal to 2n+ 2 can be employed, but, of course, are not preferred in view of their known environmental effects. Additionally, noncondensable gases such as carbon dioxide and nitrous oxide and mixtures thereof can be used and are preferred. Additionally, any other propellant capable of dispensing the concentrate known to one of ordinary skill in the art can be used. Mixtures of various propellants and diluents known to one of ordinary skill in the art are often employed to obtain the desired vapor pressure with the container. As will be seen, the propellant can be a separate propellant or can also provide the compressed gas component of the inventive composition.

Typically, the second container 30 includes enough concentrate to prepare approximately two to four batches of the composition.

To prepare the inventive composition, the first container 20 is inverted and the stem 24 is placed adjacent the stem 34 of the second container as shown in FIGURE 2. To assist in aligning the stem 24 and the stem 34 a guide 40 is provided. This guide 40 extends over a portion of the stems 24 and 34 when the stems are adjacent one another as shown in FIGURES 2 and 3. To further assist in the alignment of the valve stems, as shown in FIGURE 3, one stem can include a male configuration and the other stem can include a female configuration. When the two stems 24 and 34 are adjacent one another, pressure is applied to open the valves 22 and 32. Since the second container 30 includes a propellant such as compressed gas, concentrate is transferred from the second container to the first container. In preferred embodiments, the concentrate remains substantially free from foaming when transferred. When the transfer is complete, the pressure is released and the valves return to there normally seated and closed position. To assist in the transfer of the appropriate amount of concentrate from the second container 30 to the first container 20, the first container can include graduated markings 50 as shown in FIGURE 1. The translucency or transparency of the first container allows a user to observe the level of the contents of the first container and discontinue transfer when the appropriate level has been reached. The first container 20 can then be agitated to thoroughly mix the concentrate and diluent.

In an additional method of preparing the inventive composition, the first container 20 also includes compressed gas therein. In such an embodiment, the pressure of the second container 30 must be greater than that of the first container 20 to allow for transfer of the concentrate into the first container. Transfer of the concentrate to the first container then occurs as described above.

In an alternative method, the first container is provided with a resilient filling closure 60 as shown in FIGURE 5. This resilient filling closure 60 is shown in the closed position, but includes a passage therethrough which is normally closed due to the resiliency of the filling closure. To effect a transfer of the concentrate from the second container 30 to the first container 20, the valve stem 34 of the second container is inserted into the resilient filling closure 60, thereby opening the passage therein, and pressure is applied to effect transfer. As above, the transfer of concentrate continues until the appropriate amount of concentrate is transferred to the first container.

An alternative method of preparing the inventive composition includes providing a first container 20 having a removable cap 55 as shown in FIGURE 5. When preparing the composition, the cap is removed from the first container and diluent is added. Preferably, the diluent is chilled to about 4°C (40°F) to reduce the likelihood of foaming of the composition during preparation. The proper amount of concentrate is transferred into the open container from the second container 30. After the appropriate amount of concentrate has been transferred, a gas producing substance is added to the first container and the container is closed with the cap 55. The first container can then be agitated to mix the contents.

The gas producing substance can be in the form of a liquid, gel or solid. Preferably, the gas producing substance is an effervescing tablet of sodium bicarbonate and citric acid or sodium bicarbonate and sodium citrate.

Alternatively, the gas producing substance can be added to the first container before the concentrate is introduced. In preparing the composition, various additives can be included in either the concentrate or in the diluent, or can be added to the first container by various methods known to one of ordinary skill in the art.

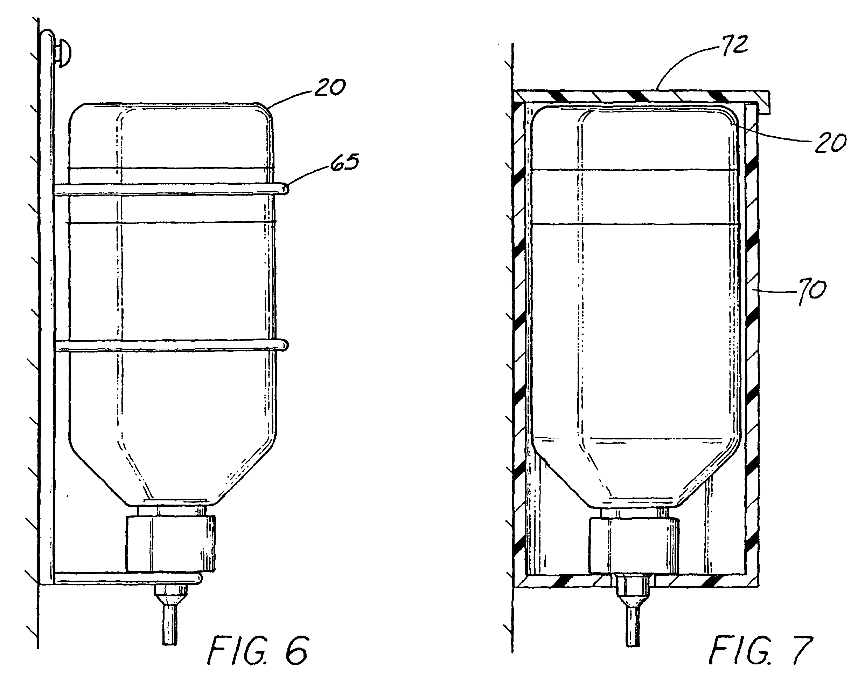

The composition is dispensed from the first container 20 through the valve 22. The valve can be actuated by downward or angular (lateral) pressure. The first container should preferably be used in the inverted position as shown in FIGURE 1. Alternative manners of attaching the dispensing container to a vertical surface are shown in FIGURES 6 and 7. FIGURE 6 shows a first container in a rack 65 which can be placed over shower head or peg-like protrusion. FIGURE 7 shows the first container 20 enclosed in a dispenser 70 that includes a removable top 72. Alternative mounting arrangements known to one of ordinary skill in the art can also be used.

The composition is dispensed from the first container as a creamy, rich foam which exhibits multiple sequential stages or levels of foam. The initial stage of foam where the utilitarian constituent is foamed by the compressed gas on dispensing has a density of about 0.15 - 0.90 g/cc. In preferred embodiments, the initial stage of foam has a density of about .15 -.30 g/cc. The second stage of foam resulting from the vaporizing post-foaming agent has a density of about 0.04 - 0.30 g/cc. Preferred embodiments exhibit a second stage of foam having a density of about .05 - .10 g/cc. Additional stages of foam are possible including a stage of foam resulting from agitation of the composition on a surface to be cleaned, thereby entraining atmospheric air in the utilitarian constituent. These multiple stages or levels of foam provide for extended foam character and less repeated use of the composition during the grooming or cleaning process.

### Examples of the Invention

Several various examples of inventive compositions suitable for use as personal care products are discussed below. All of the percentages are weight percentages.

### EXAMPLE 1

| | CONSTITUENT | % |
|---|---|---|
| **Concentrate:** | | |
| | Carbopol ETD 2020 (Thickener) | 1.50 |
| | Plantaren PS300 | 30.00 |
| | Ammonium Lauryl Sulfate, Decyl Polyglucose | |
| | Isobutane | 2.00 |
| | Isopentane | 8.00 |
| | Water | 58.50 |

| **Diluent:** | | |
|---|---|---|
| | Carbonated Water | 98.50 |
| | Triethanolamine (TEA) | 1.50 |

| **Dilution Ratio:** | | |
|---|---|---|
| | Concentrate | 33.00 |
| | Diluent | 66.00 |

The composition of Example 1 appeared thick prior to dispensing. The foam produced was lacy and voluminous. Such a composition would be suitable for use as a shampoo or similar product.

### EXAMPLE 2

| | CONSTITUENT | % |
|---|---|---|
| **Concentrate:** | | |
| | Plantaren PS200 | 30.00 |
| | Sodium Laureth Sulfate, Decyl Polyglucose | |
| | Guar Hydroxypropyl Trimonium Chloride | 1.00 |
| | (thickener/conditioning agent) | |
| | Citric Acid (acidulant) | 0.10 |
| | Isobutane | 3.00 |
| | Isopentane | 7.00 |
| | water | 58.90 |

| **Diluent:** | | |
|---|---|---|
| | Carbonated Water | 100.00 |

| **Dilution Ratio:** | | |
|---|---|---|
| | Concentrate | 30.00 |
| | Diluent | 70.00 |

The composition of Example 2 appeared thick. The foam produced was a slippery, lubricious foam. Such a composition could be used as a shaving foam.

### EXAMPLE 3

| | CONSTITUENT | % |
|---|---|---|
| **Concentrate:** | | |
| | Carbopol ETD 2020 (thickener) | 1.75 |
| | Plantaren PS300 | 25.00 |
| | Ammonium Lauryl Sulfate, Decyl Polyglucose | |
| | Polysorbate-20 (viscosity modifier) | 10.00 |
| | Triethanolamine (TEA) - (alkali to neutralize Carbopol) | 0.10 |
| | Ammonium Lauryl Sulfate | 30.00 |
| | Isobutane | 4.00 |
| | Isopentane | 5.00 |
| | water | 24.15 |

| **Diluent:** | | |
|---|---|---|
| | Water | 96.50 |
| | Sodium Bicarbonate, Sodium Citrate | 3.50 |

| **Dilution Ratio:** | | |
|---|---|---|
| | Concentrate | 50.00 |
| | Diluent | 50.00 |

The composition of Example 3 was somewhat thinner than the previous examples. The foam produced was a thick, airy, lacy foam. This composition would also be suitable for use as a shaving product.

### EXAMPLE 4

| | CONSTITUENT | % |
|---|---|---|
| **Concentrate:** | | |
| | Carbopol ETD 2020 (thickener) | 1.25 |
| | Plantaren PS300 | 20.00 |
| | Ammonium Lauryl Sulfate, Decyl Polyglucose | |
| | Hexylene Glycol (viscosity modifier) | 2.00 |
| | PEG-7 Glyceryl Cocoate (emollient) | 2.00 |
| | Dimethicone Copolyol Eicosanate - (emollient) | 3.00 |
| | Triethanolamine (TEA) - (alkali to neutralize Carbopol) | 0.10 |
| | Ammonium Lauryl Sulfate | 25.00 |
| | Polymethoxy Bicyclic Oxazolidine - (preservative) | 0.60 |
| | Isobutane | 3.00 |
| | Isopentane | 9.00 |
| | water | 34.05 |

| **Diluent:** | | |
|---|---|---|
| | Water | 98.25 |
| | Sodium Bicarbonate, Sodium Citrate | 1.75 |

| **Dilution Ratio:** | | |
|---|---|---|
| | Concentrate | 40.00 |
| | Diluent | 60.00 |

The composition of Example 4 was also somewhat thinner than the compositions of examples 1 and 2. The composition produced a soft, emollient voluminous lather. This composition would be suitable for use as a shower cleanser or the like.

### EXAMPLE 5

| | CONSTITUENT | % |
|---|---|---|
| **Concentrate:** | | |
| | Carbopol ETD 2020 | 1.60 |
| | Plantaren PS300 | 25.00 |
| | Ammonium Lauryl Sulfate, Decyl Polyglucose | |
| | Hexylene Glycol (viscosity modifier) | 1.00 |
| | PEG-7 Glyceryl Cocoate (emollient) | 1.00 |
| | Dimethicone Copolyol Eicosanate - (emollient) | 2.00 |
| | Triethanolamine (TEA) - (alkali to neutralize Carbopol) | 0.40 |
| | Ammonium Lauryl Sulfate | 15.00 |
| | Ammonium Cocoyl Isethioniate | 10.00 |
| | Polymethoxy Bicyclic Oxazolidine - (preservative) | 0.60 |
| | Isobutane | 2.00 |
| | Isopentane | 8.00 |
| | water | 33.40 |

| **Diluent:** | | |
|---|---|---|
| | Water | 98.25 |
| | Sodium Bicarbonate, Sodium Citrate | 1.75 |

| **Dilution Ratio:** | | |
|---|---|---|
| | Concentrate | 33.00 |
| | Diluent | 66.00 |

The composition of Example 5 was thin. This composition produced a soft, smooth, emollient lather also suitable for use as a shower cleanser or the like.

### EXAMPLE 6

| | CONSTITUENT | % |
|---|---|---|
| **Concentrate:** | | |
| | Citric Acid | 0.30 |
| | Plantaren PS300 | 40.00 |
| | Ammonium Lauryl Sulfate, Decyl Polyglucose | |
| | Soyamidopropyl Betaine | 15.00 |
| | Isobutane | 4.00 |
| | Isopentane | 4.00 |
| | water | 36.70 |

| **Diluent:** | | |
|---|---|---|
| | Water | 98.25 |
| | Sodium Bicarbonate, Sodium Citrate | 1.75 |

| **Dilution Ratio:** | | |
|---|---|---|
| | Concentrate | 25.00 |
| | Diluent | 75.00 |

The composition of Example 6 was thin (less than 1000 cps). This composition produced a rich, dense lather. Such a composition could be used as a shampoo.

### EXAMPLE 7

| | CONSTITUENT | % |
|---|---|---|
| **Concentrate:** | | |
| | Citric Acid (acidulant) | 0.20 |
| | Plantaren PS300 | 30.00 |
| | Ammonium Lauryl Sulfate, Decyl Polyglucose | |
| | Soyamidopropyl Betaine | 2.00 |
| | Hydroxypropyl Methylcellulose - (thickener) | 0.50 |
| | Isobutane | 3.00 |
| | Isopentane | 6.00 |
| | water | 58.30 |

| **Diluent:** | | |
|---|---|---|
| | Water | 98.25 |
| | Sodium Bicarbonate, Sodium Citrate | 1.75 |

| **Dilution Ratio:** | | |
|---|---|---|
| | Concentrate | 20.00 |
| | Diluent | 80.00 |

The composition of Example 7 was thicker than the compositions of examples 5 and 6 but thinner than those of examples 1 and 2. The composition produced a lubricious, rich lather. Such a composition could be used as a shampoo.

### EXAMPLE 8

| | CONSTITUENT | % |
|---|---|---|
| **Concentrate:** | | |
| | Carbopol ETD 2020 | 1.60 |
| | Plantaren PS300 | 25.00 |
| | Ammonium Lauryl Sulfate, Decyl Polyglucose | |
| | Hexylene Glycol (viscosity modifier) | 1.00 |
| | PEG-7 Glyceryl Cocoate (emollient) | 1.00 |
| | Dimethicone Copolyol Eicosanate - (emollient) | 2.00 |
| | Triethanolamine (TEA) - (alkali to neutralize Carbopol) | 0.40 |
| | Ammonium Lauryl Sulfate | 15.00 |
| | Ammonium Cocoyl Isethioniate | 10.00 |
| | Polymethoxy Bicyclic Oxazolidine - (preservative) | 0.60 |
| | Isobutane | 2.00 |
| | Isopentane | 8.00 |
| | water | 33.40 |

| **Diluent:** | | |
|---|---|---|
| | Water | 96.50 |
| | Sodium Bicarbonate, Sodium Citrate | 3.50 |

| **Dilution Ratio:** | | |
|---|---|---|
| | Concentrate | 33.00 |
| | Diluent | 66.00 |

The composition of Example 8 was thin (similar to that found in example 5). The composition produced a lubricious, rich lather. Such a composition could also be used as a shower cleanser.

### EXAMPLE 9

| | CONSTITUENT | % |
|---|---|---|
| **Concentrate:** | | |
| | Carbopol ETD 2020 (thickener) | 1.75 |
| | Plantaren PS300 | 25.00 |
| | Ammonium Lauryl Sulfate, Decyl Polyglucose | |
| | Polysorbate-20 (viscosity modifier) | 10.00 |
| | Triethanolamine (TEA) - (alkali to neutralize Carbopol) | 0.10 |
| | Ammonium Lauryl Sulfate | 30.00 |
| | Isobutane | 4.00 |
| | Isopentane | 5.00 |
| | water | 75.85 |

| **Diluent:** | | |
|---|---|---|
| | Water | 96.50 |
| | Sodium Bicarbonate, Sodium Citrate | 3.50 |

| **Dilution Ratio:** | | |
|---|---|---|
| | Concentrate | 30.00 |
| | Diluent | 70.00 |

The composition of Example 9 had similar thickness to those compositions of examples 3,4 and 7. The composition produced a lacy, airy lather. Such a composition would be suitable for use as a shaving product.

These examples represent a few of the possible formulations of the inventive post-foamable foam compositions and discuss only a few of the possible uses for these compositions.

While the principles of this invention have been described in connection with specific embodiments, it should be understood clearly that these descriptions are made only by way of example and are not intended to limit the scope of the invention.

## Claims

1. A composition to be dispensed from a container comprising:
(a) 0.1 - 60% by weight of a foamable, utilitarian constituent including a surface active agent selected from the group consisting of nonionic, anionic, amphoteric and cationic surfactants;
(b) 16.0 - 99.4% by weight of water;
(c) 0.50 - 24% by weight of a post-foaming agent, said post-foaming agent being a mixture of isobutane and isopentane;
(d) a compressed gas selected from (a) nitrogen, (b) argon, (c) neon, (d) krypton, (e) xenon, (f) helium, (g) carbon dioxide, (h) nitrous oxide, and (i) mixtures thereof, in an amount sufficient (i) to provide the composition, when contained, with a dispensing pressure of 169 - 514 kPa (10 - 60 psig) and (ii) to dispense the composition as a foam.

2. The composition of Claim 1 wherein the dispensing pressure of the compressed gas is about 341 kPa (about 35 psig).

3. The composition of Claim 1 wherein the vapour pressure of the post-foaming agent is such that the resulting the composition has a vapour pressure of 141.4 to 196.5 kPa (6 to 14 psig) at 32 to 38°C (90 to 100°F).

## Patentansprüche

1. Zusammensetzung zur Abgabe aus einem Behälter, umfassend:
(a) 0,1 - 60 Gew.-% eines aufschäumbaren, nützlichen Bestandteils, umfassend ein oberflächenaktives Mittel, das ausgewählt ist aus der Gruppe, die aus nicht-ionischen, anionischen, amphoteren und kationischen oberflächenaktiven Mitteln besteht;
(b) 16,0 - 99,4 Gew.-% Wasser;
(c) 0,50 - 24 Gew.-% eines verzögert schäumenden Mittels, wobei das verzögert schäumende Mittel ein Gemisch aus Isobutan und Isopentan ist;
(d) ein komprimiertes Gas, ausgewählt aus (a) Stickstoff, (b) Argon, (c) Neon, (d) Krypton, (e) Xenon, (f) Helium, (g) Kohlendioxid, (h) Distickstoffoxid, and (i) Gemischen davon, in einer Menge, die ausreicht, um (i) auf die Zusammensetzung im Behälter einen Abgabedruck von 169 - 514 kPa (10 - 60 psig) auszuüben, und (ii) die Zusammensetzung als Schaum abzugeben.

2. Zusammensetzung nach Anspruch 1, worin der Abgabedruck des komprimierten Gases etwa 341 kPa (etwa 35 psig) beträgt.

3. Zusammensetzung nach Anspruch 1, worin der Dampfdruck des verzögert schäumenden Mittels derart ist, das die endgültige Zusammensetzung einen Dampfdruck von 141,4 bis 196,5 kPa (6 bis 14 psig) bei 32 bis 38°C (90 bis 100°F) hat.

## Revendications

1. Composition à distribuer à partir d'un récipient comprenant :
(a) 0,1 - 60 % en poids d'un constituant utilitaire pouvant mousser incluant un agent tensio-actif choisi parmi des tensio-actifs non ioniques, anioniques, amphotères et canoniques;
(b) 16,0 - 99,4 % en poids d'eau;
(c) 0,50 - 24 % en poids d'un agent de moussage différé, ledit agent de moussage différé étant un mélange d'isobutane et d'isopentane;
(d) un gaz comprimé choisi parmi (a) l'azote, (b) l'argon, (c) le néon, (d) le krypton, (e) le xénon, (f) l'hélium, (g) le dioxyde de carbone, (h) l'oxyde nitreux et (i) des mélanges de ceux-ci, dans une quantité suffisante (i) pour fournir la composition, lorsqu'il est contenu, avec une pression de distribution de 169 - 514 kPa (10-60 psig) et (ii) pour distribuer la composition comme une mousse.

2. Composition selon la revendication 1, dans laquelle la pression de distribution du gaz comprimé est d'environ 341 kPa (environ 35 psig).

3. Composition selon la revendication 1, dans laquelle la pression de vapeur de l'agent de moussage différé est telle que la composition résultante présente une pression de vapeur de 141,4 à 196,5 kPa (6 à 14 psig) à de 32 à 38°C (90 à 100°F).
